(19) Europäisches Patentamt

European Patent Office

Office européen des brevets

(11) EP 2 048 589 A2

(12) **EUROPEAN PATENT APPLICATION**

(43) Date of publication:
**15.04.2009 Bulletin 2009/16**

(51) Int Cl.:
***G06F 19/00*** *(2006.01)*

(21) Application number: **08165814.8**

(22) Date of filing: **03.10.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **03.10.2007 US 997486 P**

(71) Applicant: **The Procter and Gamble Company Cincinnati, Ohio 45202 (US)**

(72) Inventors:
• **Laidig, William David**
  **Hamilton OH 45013 (US)**
• **Eike, David Michael**
  **West Chester OH 45069 (US)**

(74) Representative: **Peet, Jillian Wendy**
**Procter & Gamble Technical Centres Limited**
**Whitley Road**
**Longbenton**
**Newcastle upon Tyne**
**NE12 9TS (GB)**

(54) **Modeling systems for consumer goods**

(57) The present invention relates to modeling systems for designing and/or selecting components for use in consumer products; designing consumer products; and designing and selecting processes for making said components and said consumer products as well as the use of same.

**EP 2 048 589 A2**

**Description**

**FIELD OF THE INVENTION**

The present invention relates to modeling systems for designing and/or selecting components for use in consumer products; designing consumer products; and designing and selecting processes for making said components and said consumer products as well as the use of same.

**BACKGROUND OF THE INVENTION**

Consumer goods are typically designed and/or formulated using empirical methods or basic modeling methodologies. Such efforts are time consuming, expensive and, in the case of empirical methodologies, generally do not result in optimum designs/formulations as not all components and parameters can be considered. Furthermore, aspects of such methods may be limited to existing components. Thus there is a need for an effective and efficient methodology that obviates the short comings of such methods. The modeling systems of the present invention meet the aforementioned need and, in addition, can be used to define components, processes and superior formulations.

**SUMMARY OF THE INVENTION**

The present invention relates to modeling systems for designing and/or selecting components for use in consumer products; designing consumer products; and designing and selecting processes for making said components and said consumer products as well as the use of same.

**DETAILED DESCRIPTION OF THE INVENTION**

Definitions

As used herein "consumer products" includes, unless otherwise indicated, articles, baby care, beauty care, fabric & home care, family care, feminine care, health care, snack and/or beverage products or devices intended to be used or consumed in the form in which it is sold, and is not intended for subsequent commercial manufacture or modification. Such products include but are not limited to home décor, batteries, diapers, bibs, wipes; products for and/or methods relating to treating hair (human, dog, and/or cat), including bleaching, coloring, dyeing, conditioning, shampooing, styling; deodorants and antiperspirants; personal cleansing; cosmetics; skin care including application of creams, lotions, and other topically applied products for consumer use; and shaving products, products for treating fabrics, hard surfaces and any other surfaces in the area of fabric and home care, including: air care, car care, dishwashing, fabric conditioning (including softening), laundry detergency, laundry and rinse additive and/or care, hard surface cleaning and/or treatment, and other cleaning for consumer or institutional use; products and/or methods relating to bath tissue, facial tissue, paper handkerchiefs, and/or paper towels; tampons, feminine napkins; products and/or methods relating to oral care including toothpastes, tooth gels, tooth rinses, denture adhesives, tooth whitening; over-the-counter health care including cough and cold remedies, pain relievers, pet health and nutrition, and water purification; processed food products intended primarily for consumption between customary meals or as a meal accompaniment (non-limiting examples include potato chips, tortilla chips, popcorn, pretzels, corn chips, cereal bars, vegetable chips or crisps, snack mixes, party mixes, multigrain chips, snack crackers, cheese snacks, pork rinds, corn snacks, pellet snacks, extruded snacks and bagel chips); and coffee and cleaning and/or treatment compositions.

As used herein, the term "cleaning and/or treatment composition" includes, unless otherwise indicated, tablet, granular or powder-form all-purpose or "heavy-duty" washing agents, especially cleaning detergents; liquid, gel or paste-form all-purpose washing agents, especially the so-called heavy-duty liquid types; liquid fine-fabric detergents; hand dishwashing agents or light duty dishwashing agents, especially those of the high-foaming type; machine dishwashing agents, including the various tablet, granular, liquid and rinse-aid types for household and institutional use; liquid cleaning and disinfecting agents, including antibacterial hand-wash types, cleaning bars, mouthwashes, denture cleaners, car or carpet shampoos, bathroom cleaners; hair shampoos and hair-rinses; shower gels and foam baths and metal cleaners; as well as cleaning auxiliaries such as bleach additives and "stain-stick" or pre-treat types.

As used herein, "component of a consumer product" encompasses consumer product components and packaging.

As used herein, the term "situs" includes paper products, fabrics, garments and hard surfaces.

As used herein, the articles a and an when used in a claim, are understood to mean one or more of what is claimed or described.

Unless otherwise noted, all component or composition levels are in reference to the active level of that component or composition, and are exclusive of impurities, for example, residual solvents or by-products, which may be present in commercially available sources.

All percentages and ratios are calculated by weight unless otherwise indicated. All percentages and ratios are calculated based on the total composition unless otherwise indicated.

It should be understood that every maximum numerical limitation given throughout this specification includes every lower numerical limitation, as if such lower numerical limitations were expressly written herein. Every minimum numerical limitation given throughout this specification will include every higher numerical limitation, as if such higher numerical limitations were expressly written herein. Every numerical range given throughout this specification will include every narrower numerical range that falls within such broader numerical range, as if such narrower numerical ranges were all expressly written herein.

Modeling Methods

In a first aspect, Applicant's modelling method comprises:

a.) calculating, for a system comprising at least one species, having at least one species characteristic, at least one thermodynamic property, physical property and/or molecular descriptor of said system using said at least one species characteristic;
b.) optionally, using the thermodynamic property, physical property and/or molecular descriptor calculated in a.) to:

(i) calculate at least one additional thermodynamic property, physical property and/or molecular descriptor of said system; and/or
(ii) refine the thermodynamic property, physical property and/or molecular descriptor calculated in a.); and

c.) optionally repeating a.) through b.) one or more times; and
d.) using the thermodynamic property, physical property and/or molecular descriptor obtained from any of a.) through c.) to design and/or select a component for use in consumer product; design a consumer product; design and/or select a process for making said component and/or said consumer product.

In one aspect, such method comprises calculating using at least two system parameters of a system comprising at least one species and at least one phase, wherein at least one of said parameters is a species characteristic, at least one thermodynamic property, physical property and/or molecular descriptor of said system.

In one aspect, such method comprises using the thermodynamic property, physical property and/or molecular descriptor calculated in a.) to calculate at least one thermodynamic property, physical property and/or molecular descriptor of said system, or to refine the thermodynamic property, physical property and/or molecular descriptor calculated in a.).

In one aspect, of such method a.) through b.) are repeated one or more times.

In one aspect of such method, said calculation of said at least one thermodynamic property, physical property and/or molecular descriptor of said system is based on from 1 to about 1000, from 1 to about 500, or even from 2 to about 150 system parameters.

In one aspect of such method, d.) comprises comparing said thermodynamic property, physical property and/or molecular descriptor obtained from any of a.) through c.) with one or more additional thermodynamic properties, physical properties and/or molecular descriptors calculated in accordance with any of the calculation steps a.) through c.) of said method.

In one aspect of such method, said at least one species characteristic used to calculate said at least one thermodynamic property, physical property and/or molecular descriptor of said system comprises at least one species molecular state of said species.

In one aspect of such method, said species molecular state comprises a total charge, an electronic state, and a molecular structure wherein said molecular structure comprises one or more elements independently selected from the group consisting of:

a.) number of atoms, atom types, and/or isotopes;
b.) mole fractions of atoms, atom types, and/or isotopes;
c.) molecular connectivity; and
d.) one or more sets of 3D atomic coordinates.

In one aspect, the calculation of said at least one thermodynamic property, physical property and/or molecular descriptor of said system employs one or more system parameters selected from the group consisting of:

3

a.) a species molecular state;
b.) phase composition;
c.) system temperature;
d.) system pressure;
e.) system and/or phase external magnetic field;
f.) system and/or phase external electric field;
g.) system external gravitational field;
h.) phase dielectric constant;
i.) atomic/molecular cavity size;
j.) phase interface topology; and/or
k.) a species thermodynamic property.

In one aspect of such method, the calculation of said at least one thermodynamic property, physical property and/or molecular descriptor of said system employs one or more system parameters selected from the group consisting of:

a.) a species molecular state;
b.) phase composition;
c.) system temperature;
d.) phase dielectric constant;
e.) atomic/molecular cavity size; and/or
f.) a species thermodynamic property.

In one aspect of such method, wherein said phase composition comprises one or more of the following elements: species mole fraction, species spatial probability, phase pH, phase ionic strength and said species thermodynamic property is selected from the group consisting of:

a.) free energy of phase change for a pure species;
b.) enthalpy of phase change for a pure species;
c.) entropy of phase change for a pure species;
d.) energy of phase change for a pure species;
e.) a pure species phase transition temperature;
f.) energy of pure species in vacuum;
g.) entropy of pure species in vacuum;
h.) a pure species vapor pressure;
i.) a pure phase constant pressure heat capacity; and
j.) a pure phase constant volume heat capacity.

In one aspect of such method:

a.) said thermodynamic and/or physical property is selected from the group consisting of:

(i) system, phase, species and/or subspecies free energy;
(ii) system, phase, species and/or subspecies enthalpy;
(iii) species and/or subspecies chemical potential;
(iv) species and/or subspecies activity coefficient;
(v) the equilibrium concentration of a species;
(vi) pure species density;
(vii) pure species viscosity;
(viii) pure species vapor pressure;
(ix) species pKa;
(x) reaction free energy barrier;
(xi) spatial distribution of a species in an inhomogeneous phase;
(xii) species enthalpy of adsorption;
(xiii) species free energy of adsorption;
(xiv) polymer swelling degree; and/or
(xv) absorption, distribution, metabolism, excretion prediction;

b.) said molecular descriptor is selected from the group consisting of;

(i) system, phase, and/or species sigma-moments;
(ii) system, phase, species and/or subspecies sigma profiles;
(iii) species and/or subspecies cavity volume;
(iv) species and/or subspecies cavity surface area;
(v) moments of cavity charge-density;
(vi) sigma-profile similarity index;
(vii) screening charge distribution based charged partial surface area descriptors; and/or
(viii) ligand scoring/fit functions.

In one aspect of such method the calculated at least one:

a.) said thermodynamic and/or physical property is selected from the group consisting of:

(i) system, phase, species and/or subspecies free energy;
(ii) system, phase, species and/or subspecies enthalpy;
(iii) species and/or subspecies chemical potential;
(iv) species and/or subspecies activity coefficient;
(v) the equilibrium concentration of a species;
(vi) pure species density;
(vii) pure species viscosity;
(viii) pure species vapor pressure;
(ix) species pKa;
(x) reaction free energy barrier;
(xi) spatial distribution of a species in an inhomogeneous phase;
(xii) species enthalpy of adsorption;
(xiii) species free energy of adsorption;
(xiv) polymer swelling degree; and/or
(xv) absorption, distribution, metabolism, excretion prediction;

b.) said molecular descriptor is selected from the group consisting of;

(i) system, phase, and/or species sigma-moments;
(ii) system, phase, species and/or subspecies sigma profiles;
(iii) species and/or subspecies cavity volume;
(iv) species and/or subspecies cavity surface area;
(v) moments of cavity charge-density;
(vi) sigma-profile similarity index;
(vii) screening charge distribution based charged partial surface area descriptors; and/or
(viii) ligand scoring/fit functions.

In one aspect of such method, the calculated at least one:

a.) said thermodynamic and/or physical property is selected from the group consisting of:

(i) system, phase, species and/or subspecies free energy;
(ii) system, phase, species and/or subspecies enthalpy;
(iii) species and/or subspecies chemical potential;
(iv) species and/or subspecies activity coefficient;
(v) the equilibrium concentration of a species;
(vi) pure species vapor pressure;
(vii) species pKa;
(viii) reaction free energy barrier;
(ix) spatial distribution of a species in an inhomogeneous phase;
(x) species enthalpy of adsorption;
(xi) species free energy of adsorption; and/or
(xii) absorption, distribution, metabolism, excretion prediction;

b.) said molecular descriptor is selected from the group consisting of;

(i) system, phase, and/or species sigma-moments;
(ii) system, phase, species and/or subspecies sigma profiles;
(iii) species and/or subspecies cavity volume;
(iv) species and/or subspecies cavity surface area;
(v) sigma-profile similarity index; and/or
(vi) screening charge distribution based charged partial surface area descriptors.

In one aspect of such method, the calculated at least one:

a.) said thermodynamic and/or physical property is selected from the group consisting of:

(i) system, phase, species and/or subspecies free energy;
(ii) system, phase, species and/or subspecies enthalpy;
(iii) species and/or subspecies chemical potential;
(iv) the equilibrium concentration of a species;
(v) pure species vapor pressure;
(vi) species pKa;
(vii) reaction free energy barrier; and/or
(viii) spatial distribution of a species in an inhomogeneous phase;

b.) said molecular descriptor is selected from the group consisting of;

(i) system, phase, and/or species sigma-moments;
(ii) system, phase, species and/or subspecies sigma profiles;
(iii) species and/or subspecies cavity volume;
(iv) species and/or subspecies cavity surface area; and/or
(v) sigma-profile similarity index.

In one aspect of such method, the method comprises using the thermodynamic property, physical property and/or molecular descriptor calculated in a.) to:

(i) calculate at least one additional thermodynamic property, physical property and/or molecular descriptor of said system; and/or
(ii) refine the thermodynamic property, physical property and/or molecular descriptor calculated in a.).

In one aspect of the method, Steps a.) through c.) are repeated at least once.
In one aspect of the method Step a.) comprises determining numerical parameters for at least one of said species and, if said determination is not sufficient to produce said thermodynamic property, physical property and/or molecular descriptor;

a.) repeating such determination using an input comprising the results of said determination; and/or
b.) calculating, in accordance with a molecular thermodynamic theory, using an input comprising said numerical parameters, an output; and if said determination and/or calculation is not sufficient to produce said thermodynamic property, physical property and/or molecular descriptor;
c.) repeating one or more times, a.) and/or b.) using, said numerical parameters and/or said output.
In one aspect of the method said determination comprises obtaining existing numerical parameters and/or calculating said numerical parameters.

In one aspect of the method, numerical parameters are selected from the group comprising screening charge density at dielectric boundary, histogram of surface area with respect to screening charge density, molecular surface, molecular volume, collision diameter, interaction energy, and/or optimized 3D structure.
In one aspect of the method, said calculation comprises molecular modelling, molecular simulation, quantum chemistry calculation, continuum solvation analysis, and/or molecular thermodynamic modelling.
In one aspect of the method, said molecular thermodynamic theory is selected from the group consisting of COSMO-RS theory, SAFT theory, quasi-chemical theory, lattice fluid theory, mean-field theory, and combinations thereof.
In one aspect of the method, the species are not odorants.
In one aspect of the method, the species are not cosmetic auxiliaries.
In one aspect of the method the species are not aroma substances.

In one aspect of the method, when the species is a protein and thermodynamic property is not pKa.

In one aspect of the method, using the thermodynamic property, physical property and/or molecular descriptor obtained from any of a.) through c.) to design and/or select a component for use in consumer product; design a consumer product; design and/or select a process for making said component and/or said consumer product does not employ regression.

In one aspect of the method, using the thermodynamic property, physical property and/or molecular descriptor obtained from any of a.) through c.) to design and/or select a component for use in consumer product; design a consumer product; design and/or select a process for making said component and/or said consumer product does not employ regression of odorant data.

In one aspect of the method, using the thermodynamic property, physical property and/or molecular descriptor obtained from any of a.) through c.) to design and/or select a component for use in consumer product; design a consumer product; design and/or select a process for making said component and/or said consumer product does not employ regression of cosmetic auxiliary data.

In one aspect of the method, using the thermodynamic property, physical property and/or molecular descriptor obtained from any of a.) through c.) to design and/or select a component for use in a consumer product; to design a consumer product; design and/or select a process for making said component and/or said consumer product does not employ regression of aroma substance data.

In one aspect, computationally calculated thermodynamic properties (including but not limited to solubility, heats and free energies of formation and mixing; thermal expansion; vapor pressure; specific heat at constant pressure; specific heat at constant volume; dynamic viscosity; kinematic viscosity; thermal conductivity; thermal diffusivity; volumetric thermal expansion coefficient; enthalpy of fusion & vaporization; entropy; Gibbs free energy; pressure; electrical potential of the reaction; equilibrium constant (e.g. acid dissociation constant; binding constant; chemical equilibrium; dissociation constant; distribution coefficient; reaction quotient; solubility equilibrium; stability constant; thermodynamic equilibrium; vapor-liquid equilibrium, liquid-liquid equilibrium); gas and liquid phase ionization potentials; boiling and melting point to predict performance properties of beauty consumer products including, without limitation unless otherwise indicated, lipsticks, lip paints, lip gloss, lip softeners, eye mascaras, eye shadows, eye pencils, eye lash thickeners, face cosmetics (rouges, foundations, creams, coverers), skin creams, lotions & milks, skin cleansers, skin whiteners, skin feel, skin moisturizers, exfoliants, anti-acne agents, sun protectors, hair sprays, hair volumizers and bodifiers, hair gels, hair mousses, hair waxes, hair tonic, shampoos, conditioners, soap bars, body washes, shine agents, hair dyes, bleaches, perming agents, straightening agents, sun protectants, anti-dandruff shampoos, antiperspirants, deodorants, toothpaste, and tooth whitening agents.

In one aspect, computationally calculated thermodynamic properties are used to predict health and beauty consumer performance attributes including but without limitation hair shine, hair condition, skin feel, skin whiteness, hair volume/body, tooth whiteness, lip softness, lip fullness, eye lash thickness, breath freshness, skin condition, skin tone, skin naturalness, etc.); sensory areas (e.g., including without limitation touch taste, smell, visual, sound); consumer sensory attributes (e.g., including without limitation shampoo creaminess, cream whiteness, toothpaste flavor (e.g., minty-ness), lather creaminess/whiteness/rinseability, styling gel stickiness/hold, mascara durability, foundation greasiness, lipstick durability, etc.); product stability (e.g., including without limitation light stability, temperature stability, stability to metal ions, etc.); package performance attributes (e.g., including without limitation ease of actuation, ease of grip, package breatheability, spray characteristics (low versus high particle size, wet versus dry, etc.), etc.), and package stability (e.g., including without limitation erosion stability, pressure stability, etc.).

In one aspect of the method, the method may comprise a combination of aspects of Applicants' invention that are described above.

A component for use in consumer product, a consumer product, or process for making said component and/or said consumer product designed or selected in accordance with any aspect of the foregoing method.

Consumer Products

As taught by the present specification, including the examples included herein, the method disclosed herein may be used to design and/or select a component for use in consumer product, a consumer product, or process for making said component and/or said consumer product. Suitable adjunct materials are listed below.

Adjunct Materials

While not essential for the purposes of the present invention, the non-limiting list of adjuncts illustrated hereinafter are suitable for use in the instant compositions and may be desirably incorporated in certain embodiments of the invention, for example to assist or enhance cleaning performance, for treatment of the substrate to be cleaned, or to modify the aesthetics of the cleaning composition as is the case with perfumes, colorants, dyes or the like. It is understood that such adjuncts are in addition to the dye conjugate and optional stripping agent components of Applicants' compositions. The precise nature of these additional components, and levels of incorporation thereof, will depend on the physical form of the composition and the nature of the cleaning operation for which it is to be used. Suitable adjunct materials include, but are not limited to, surfactants, builders, chelating agents, dye transfer inhibiting agents, dispersants, enzymes, and enzyme stabilizers, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, structurants, hydrotropes, processing aids, solvents and/or pigments. In addition to the disclosure below, suitable examples of such other adjuncts and levels of use are found in U.S. Patent Nos. 5,576,282, 6,306,812 B1 and 6,326,348 B1 that are incorporated by reference.

As stated, the adjunct ingredients are not essential to Applicants' compositions. Thus, certain embodiments of Applicants' compositions do not contain one or more of the following adjuncts materials: surfactants, builders, chelating agents, dye transfer inhibiting agents, dispersants, enzymes, and enzyme stabilizers, catalytic materials, bleach activators, hydrogen peroxide, sources of hydrogen peroxide, preformed peracids, polymeric dispersing agents, clay soil removal/anti-redeposition agents, brighteners, suds suppressors, dyes, perfumes, structure elasticizing agents, fabric softeners, carriers, hydrotropes, processing aids, solvents and/or pigments. However, when one or more adjuncts are present, such one or more adjuncts may be present as detailed below: Bleaching Agents - Bleaching agents other than bleaching catalysts include photobleaches, bleach activators, hydrogen peroxide, sources of hydrogen peroxide, preformed peracids. Examples of suitable bleaching agents include anhydrous sodium perborate (mono or tetra hydrate), anhydrous sodium percarbonate, tetraacetyl ethylene diamine, nonanoyloxybenzene sulfonate, sulfonated zinc phtalocyanine and mixtures thereof.

When a bleaching agent is used, the compositions of the present invention may comprise from about 0.1% to about 50% or even from about 0.1% to about 25% bleaching agent by weight of the subject cleaning composition.

Surfactants - The compositions according to the present invention may comprise a surfactant or surfactant system wherein the surfactant can be selected from nonionic surfactants, anionic surfactants, cationic surfactants, ampholytic surfactants, zwitterionic surfactants, semi-polar nonionic surfactants and mixtures thereof.

The surfactant is typically present at a level of from about 0.1% to about 60%, from about 1% to about 50% or even from about 5% to about 40% by weight of the subject composition.

Builders - The compositions of the present invention may comprise one or more detergent builders or builder systems. When a builder is used, the subject composition will typically comprise at least about 1%, from about 5% to about 60% or even from about 10% to about 40% builder by weight of the subject composition.

Builders include, but are not limited to, the alkali metal, ammonium and alkanolammonium salts of polyphosphates, alkali metal silicates, alkaline earth and alkali metal carbonates, aluminosilicate builders and polycarboxylate compounds. ether hydroxypolycarboxylates, copolymers of maleic anhydride with ethylene or vinyl methyl ether, 1, 3, 5-trihydroxy benzene-2, 4, 6-trisulphonic acid, and carboxymethyloxysuccinic acid, the various alkali metal, ammonium and substituted ammonium salts of polyacetic acids such as ethylenediamine tetraacetic acid and nitrilotriacetic acid, as well as polycarboxylates such as mellitic acid, succinic acid, citric acid, oxydisuccinic acid, polymaleic acid, benzene 1,3,5-tricarboxylic acid, carboxymethyloxysuccinic acid, and soluble salts thereof.

Chelating Agents - The compositions herein may contain a chelating agent. Suitable chelating agents include copper, iron and/or manganese chelating agents and mixtures thereof.

When a chelating agent is used, the composition may comprise from about 0.1 % to about 15% or even from about 3.0% to about 10% chelating agent by weight of the subject composition.

Dye Transfer Inhibiting Agents - The compositions of the present invention may also include one or more dye transfer inhibiting agents. Suitable polymeric dye transfer inhibiting agents include, but are not limited to, polyvinylpyrrolidone polymers, polyamine N-oxide polymers, copolymers of N-vinylpyrrolidone and N-vinylimidazole, polyvinyloxazolidones and polyvinylimidazoles or mixtures thereof.

When present in a subject composition, the dye transfer inhibiting agents may be present at levels from about 0.0001 % to about 10%, from about 0.01 % to about 5% or even from about 0.1 % to about 3% by weight of the composition.

Dispersants - The compositions of the present invention can also contain dispersants. Suitable water-soluble organic materials include the homo- or co-polymeric acids or their salts, in which the polycarboxylic acid comprises at least two carboxyl radicals separated from each other by not more than two carbon atoms.

Enzymes - The compositions can comprise one or more enzymes which provide cleaning performance and/or fabric care benefits. Examples of suitable enzymes include, but are not limited to, hemicellulases, peroxidases, proteases, cellulases, xylanases, lipases, phospholipases, esterases, cutinases, pectinases, mannanases, pectate lyases, keratanases, reductases, oxidases, phenoloxidases, lipoxygenases, ligninases, pullulanases, tannases, pentosanases, malanases, β-glucanases, arabinosidases, hyaluronidase, chondroitinase, laccase, and amylases, or mixtures thereof. A typical combination is an enzyme cocktail that comprises a protease, lipase, cutinase and/or cellulase in conjunction with amylase.

When present in a cleaning composition, the aforementioned adjunct enzymes may be present at levels from about 0.00001 % to about 2%, from about 0.0001 % to about 1% or even from about 0.001% to about 0.5% enzyme protein by weight of the composition.

Enzyme Stabilizers - Enzymes for use in detergents can be stabilized by various techniques. The enzymes employed herein can be stabilized by the presence of water-soluble sources of calcium and/or magnesium ions in the finished compositions that provide such ions to the enzymes. In case of aqueous compositions comprising protease, a reversible protease inhibitor can be added to further improve stability.

Catalytic Metal Complexes - Applicants' compositions may include catalytic metal complexes. One type of metal-containing bleach catalyst is a catalyst system comprising a transition metal cation of defined bleach catalytic activity, such as copper, iron, titanium, ruthenium, tungsten, molybdenum, or manganese cations, an auxiliary metal cation having little or no bleach catalytic activity, such as zinc or aluminium cations, and a sequestrate having defined stability constants for the catalytic and auxiliary metal cations, particularly ethylenediaminetetraacetic acid, ethylenediaminetetra (methylenephosphonic acid) and water-soluble salts thereof. Such catalysts are disclosed in U.S. 4,430,243.

If desired, the compositions herein can be catalyzed by means of a manganese compound. Such compounds and levels of use are well known in the art and include, for example, the manganese-based catalysts disclosed in U.S. 5,576,282.

Cobalt bleach catalysts useful herein are known, and are described, for example, in U.S. 5,597,936; U.S. 5,595,967. Such cobalt catalysts are readily prepared by known procedures, such as taught for example in U.S. 5,597,936, and U.S. 5,595,967.

Compositions herein may also suitably include a transition metal complex of a macropolycyclic rigid ligand - abbreviated as "MRL". As a practical matter, and not by way of limitation, the compositions and processes herein can be adjusted to provide on the order of at least one part per hundred million of the active MRL species in the aqueous washing medium, and will typically provide from about 0.005 ppm to about 25 ppm, from about 0.05 ppm to about 10 ppm, or even from about 0.1 ppm to about 5 ppm, of the MRL in the wash liquor.

Suitable transition-metals in the instant transition-metal bleach catalyst include, for example, manganese, iron and chromium. Suitable MRL's include 5,12-diethyl-1,5,8,12-tetraazabicyclo[6.6.2]hexadecane.

Suitable transition metal MRLs are readily prepared by known procedures, such as taught for example in WO 00/32601, and U.S. 6,225,464.

Solvents - Suitable solvents include water and other solvents such as lipophilic fluids. Examples of suitable lipophilic fluids include siloxanes, other silicones, hydrocarbons, glycol ethers, glycerine derivatives such as glycerine ethers, perfluorinated amines, perfluorinated and hydrofluoroether solvents, low-volatility nonfluorinated organic solvents, diol solvents, other environmentally-friendly solvents and mixtures thereof.

Processes of Making Cleaning and/or Treatment Compositions

The cleaning compositions of the present invention can be formulated into any suitable form and prepared by any process chosen by the formulator, non-limiting examples of which are described in Applicants examples and in U.S. 5,879,584; U.S. 5,691,297; U.S. 5,574,005; U.S. 5,569,645; U.S. 5,565,422; U.S. 5,516,448; U.S. 5,489,392; U.S. 5,486,303 all of which are incorporated herein by reference.

Method of Use

The consumer products of the present invention may be used in any conventional manner. In short, they may be used in the same manner as consumer products that are designed and produced by conventional methods and processes. For example, cleaning and/or treatment compositions of the present invention can be used to clean and/or treat a situs inter alia a surface or fabric. Typically at least a portion of the situs is contacted with an embodiment of Applicants' composition, in neat form or diluted in a wash liquor, and then the situs is optionally washed and/or rinsed. For purposes of the present invention, washing includes but is not limited to, scrubbing, and mechanical agitation. The fabric may comprise any fabric capable of being laundered in normal consumer use conditions. Cleaning solutions that comprise the disclosed cleaning compositions typically have a pH of from about 5 to about 10.5. Such compositions are typically employed at concentrations of from about 500 ppm to about 15,000 ppm in solution. When the wash solvent is water, the water temperature typically ranges from about 5 °C to about 90°C and, when the situs comprises a fabric, the water to fabric mass ratio is typically from about 1:1 to about 100:1.

**EXAMPLES**

**Example 1:** Predicting Maximum Brightener Solubility in Liquid Laundry Detergent

To estimate the solubility of brightener salt (Tinopal DMS-X, Ciba Specialty Chemicals, Basel, Switzerland) in a range of liquid laundry formulations, a model of liquid laundry detergent (HDL) is created. Such model is used to formulate HDLs having improved brightener solubility and thus containing the higher desired levels of brightener. The input for this method is a formula card listing weight percent of all formula ingredients. The weight percents of key formula ingredients are taken. The key formula ingredients are:

> a.) surfactants: alkyl ethoxy sulphate (AES), methyl-branched alkyl sulphate (AS), linear alkyl benzene sulphonate (LAS), alkyl poly-ethoxy alcohol (NI)
> b.) builders: citric acid, alkyl carboxylic acid (fatty acid)
> c.) stabilizers: borax, calcium formate, ethanol, 1,2-propanediol, diethylene glycol, mono-ethanolamine (MEA), sodium hydroxide, sodium formate, water

Based on the molecular weights of the key ingredients, weight percent is converted to mole fraction. Next, the mole fractions of the species used in the model are determined from the mole fractions of the key ingredients using the following rules:

> a.) Only the head group plus the first methyl group of surfactants and fatty acid are considered. LAS is modelled as 4-methyl benzene sulphonate. AES is modelled as methyl bis(ethoxy) sulphate. AS is modelled as methyl sulphate. NI is modelled as methyl alcohol, 8 times ethoxylated. Fatty acid is assumed to be fully deprotonated, and is modelled as methyl carboxylate.
> b.) Borax dissociates and yields four moles of boric acid for every mole of borax. Boric acid reacts with citric acid to yield a boric acid-citric acid dianion species. Boric acid is in equilibrium with borate. Citric acid is in equilibrium with citrate, mono-acidic citrate, and di-acidic citrate. Borate reacts with 1,2-propanediol to yield a borate-diol ester. The borate-diol ester further reacts with 1,2-propanediol to yield a borate-diol di-ester. Based on the initial concentrations of boric acid, citric acid, and 1,2-propanediol, the final concentrations of boric acid, borate, citric acid, citrate, mono-acidic citrate, di-acidic citrate, boric acid-citric acid complex, borate-diol monoester, and borate-diol di-ester are solved for. The equilibrium constants are known, standard $pK_a$ values for boric acid and citric acid are used, and pH is fixed at 8.5.
> c.) Sodium formate dissociates and yields one mole of formate for every mole of sodium formate. Calcium formate dissociates and yields on mole of calcium cation and two moles of formate for every mole of calcium formate.
> d.) MEA is in equilibrium with protonated MEA. Every mole of MEA yields 0.9 moles of protonated MEA and 0.1 moles of neutral MEA.
> e.) One mole of sodium is present for every mole of excess negative charge in the system. Sodium hydroxide dissociates and yields one mole of water for every mole of sodium hydroxide.

The final list of model species comprises 4-methyl benzene sulphonate (LAS head); methyl bis(ethoxy) sulphate (AES head); methyl sulphate (AS head); methyl alcohol, 8 times ethoxylated (NI head); methyl carboxylate (fatty acid head); citrate; mono-acidic citrate; boric acid; borate; boric-citrate ester; 1,2-propanediol; borate-diol monoester; borate-diol diester; ethanol; diethylene glycol; water; neutral monoethanolamine; protonated monoethanolamine; sodium ion; calcium ion; formate; and brightener salt ion. Species are drawn using the Spartan '06 software (Wavefunction, Inc., Irvine, CA, USA) and geometry optimization and conformer selection are performed using Spartan '06. For all calculations, a single conformer is used for all species. The lowest energy conformer is selected as the main conformer. Turbomole (distributed by COSMOlogic GmbH & Co. KG, Leverkusen, Germany) is used to run a COSMO analysis on all species and create screening charge density profiles. Temperature and species mole fractions are input into the COSMOtherm software (COSMOlogic GmbH & Co. KG, Leverkusen, Germany), which calculates the chemical potential of all species using the COSMO-RS method. The entered mole fraction of brightener salt anion is zero which yields infinite dilution chemical potential for the brightener anion. It is assumed that the maximum solubility of brightener salt is low enough that infinite dilution chemical potential is equal to the finite chemical potential of the brightener salt anion. Using the COSMOtherm software, infinite dilution chemical potential of brightener salt anion and sodium are also determined in pure water to serve as a reference chemical potential. Activity coefficients for sodium and brightener salt are calculated according to

$$\ln \gamma_i = \frac{\mu_i - \mu_i^{ref}}{RT}$$

where $\gamma_i$ is the activity coefficient for species $i$, $\mu_i$ is full formula chemical potential for species $i$, $\mu_i^{ref}$ is the reference chemical potential for species $i$, R is the universal gas constant and T is temperature. An activity coefficient correction based on the ionic strength is added calculated according to a variant of the Debye-Hückel law, given as

$$\ln \gamma_i^{DH} = -Az_i^2 \left[ \frac{2}{\rho} \ln\left(1 + \rho I^{1/2}\right) + I^{1/2} \left( \frac{1 - 2I / z_i^2}{1 + \rho I^{1/2}} \right) \right]$$

$$I = 0.5 \sum_i x_i z_i^2$$

where $\gamma_i^{DH}$ is the ionic strength activity coefficient correction, A and p are constants, $x_i$ is the mole fraction of species $i$, $z_i$ is the charge of species $i$, and I is the ionic strength of the formula. The constant A is fixed at 2.917, which is usual for water at 25 °C. The parameter p is set to 0.8 based on empirical tuning. Maximum solubility of brightener is calculated according to

$$\ln x_{\pm} = -\frac{\Delta G_{diss}}{\nu RT} - \ln \gamma_{\pm}$$

$$x_{\pm} = \left( x_c^{vc} x_a^{va} \right)^{1/\nu} \qquad \gamma_{\pm} = \left( \gamma_c^{vc} \gamma_a^{va} \right)^{1/\nu}$$

$$\nu = \nu a + \nu c$$

where $x_{\pm}$ is the maximum solubility average mole fraction of the salt, $\gamma_{\pm}$ is the average activity coefficient, and $\Delta G_{diss}$ is the standard Gibbs free energy change of dissolution for the salt. All average quantities are defined as a weighted geometric mean with weights based on the stoichiometric dissolution coefficients, va and vc. The stoichiometric coefficients are taken from the dissolution equation of a salt, written as

$$A_{va} C_{vc} \leftrightarrow \nu a A^{za} + \nu c C^{zc}$$

where A and C represent the anion and cation species, respectively. The charges of the dissociated anion and cation are represented as za and zc, respectively. The free energy of dissolution value is empirically determined by fitting predicted maximum solubility values to analytically measured maximum solubility values for a training set of formulas.

**Example 2:** Predicting acid dissociation constants to generate accurate, consumer-relevant representations of molecular structure to enable accurate performance prediction of e.g. lipsticks, lip paints, lip gloss, lip softeners, eye mascaras, eye shadows, eye pencils, eye lash thickeners, face cosmetics (rouges, foundations, creams, coverers), skin creams, lotions & milks, skin cleansers, skin whiteners, skin feel, skin moisturizers, exfoliants, anti-acne agents, sun protectors, hair sprays, hair volumizers and bodifiers, hair gels, hair mousses, hair waxes, hair tonic, shampoos, conditioners, soap bars, body washes, shine agents, hair dyes, bleaches, perming agents, straightening agents, sun protectants, anti-dandruff shampoos, antiperspirants, deodorants, toothpaste, and tooth whitening agents. To estimate the $pK_a$ of a molecule the following steps are performed:

a) A 3D molecular model of the fully protonated molecule is sketched into Spartan '06 (Wavefunction Inc., Irvine, CA) and a low-energy conformer is selected by the following Spartan '06 procedure:

1. Using the MMFF force-field and the default conformational searching procedure locate the low-energy MMFF conformers for this molecule;

2. Reminimize this conformer list at the PM3 theory level.;

3. Select the lowest-energy PM3 reminimized conformer as the lowest-energy conformer.

b) Analogous models are constructed for all potential ionized forms of the molecule.

c) For all 3D models from Steps a) and b), the structures are reoptimized at the gas phase BP86/TZVP theory level in TURBOMOLE (COSMOlogic GmbH & Co. KG, Leverkusen, Germany) and the total gas phase energies are stored.

d) For all 3D models from Steps a) and b), the structures are reoptimized at the BP86/TZVP theory level including COSMO implicit solvation with infinite dielectric in TURBOMOLE and the corresponding COSMO files are generated and stored.

e) The COSMO files and gas phase energies for the fully protonated molecule, one of the singly ionized forms of the molecule, and water plus the system temperature are input to COSMOtherm (COSMOlogic GmbH & Co. KG, Leverkusen, Germany) and the $pK_a$ is estimated using the equation

$$pK_a = A\frac{\Delta G_{diss}}{RT\ln(10)} + B$$

Where $\Delta G_{diss}$ is the $G_{tot}(A-) - G_{tot}(AH)$ for the acid AH, T is the temperature, R is the gas constant and A and B are parameters best-fit to reproduce a large training set of experimental $pK_a$'s in water. Specific values used for A and B are A = -0.61255 mol/kcal and B = -118.74514 (see "First Principles Calculations of Aqueous pKa Values for Organic and Inorganic Acids Using COSMO-RS Reveal an Inconsistency in the Slope of the pKa Scale", A. Klamt, F. Eckert, M. Diedenhofen and M. Beck, J. Phys. Chem. A 107, 9380 (2003) and the COSMOtherm Manual Version C2.1 Release 01.05 for more details).

f) Step d) is repeated for any additional singly ionized forms of the molecule.

g) Step d) is repeated for all pairs of singly and doubly ionized molecules where the doubly ionized form is generated from a single ionization of the singly ionized form.

h) Step f) is repeated for any doubly or higher ionized forms of the molecule and all higher ionized forms generated by removing an additional proton from the doubly or higher ionized form.

i) The set of one or more acid dissociation constants calculated in steps d through g are used to estimate the concentrations of each ionized form at typical molecule usage pH using the coupled set of equations.

The resulting acid dissociation constant data is used as input for more accurately predicting consumer-relevant performance under consumer usage conditions. Such a model is used to screen new materials for use in the specific beauty-related performance area.

**Example 3:** Solubility of styling mousse ingredients in pressurized can coatings. Excessive ingredient solubility in can linings can lead to film rupture and can corrosion. Identifying the problem ingredients is the first step in improving can stability. To do this the following procedure is employed:

a) A specific mousse formulation and can lining composition which when used together are known to lead to can corrosion during extended storage are identified, and their formula ingredients determined;

b) One or more alternate mousse formulations which can not show the degradation problem with the can lining composition from step a) are identified and their constituent ingredients determined;

c) One or more can lining compositions that do not show degradation with the mousse formulation in step a) are identified and their constituent ingredients determined. Additionally, these can lining compositions should be stable relative to the mousse formulations in step b);

d) The ingredients in the problem and the non-problem mousse formulations are compared and a list of unique ingredients and/or ingredients that exist in very different concentrations between these 2 formula classes (>10x) is assembled;

e) Ionic ingredients are removed from the list produced step d) due to their limited solubility in typically can liner formulations;

f) For all ingredients from the list in step e), gas-phase energies and COSMO files are determined using the procedure outlined in Example 2 steps a), c) and d);

g) Gas-phase energies and COSMO files for the ingredients in the can linings specified in steps a) and c) are computed following the procedure above in steps e) - g). If any ingredients are polymers the procedure in Example 2 step a) is modified as follows:

1. For homopolymers the polymer is represented by an average monomeric unit where dangling bonds are terminated with hydrogen atoms;

2. For block copolymers the polymer is represented by two or more average monomeric units for each block where dangling bonds are terminated with hydrogen atoms. The copolymer is considered to be a weighted mixture of these molecules in the ratio equal to the ratio of the copolymer blocks in the physical polymer;

3. One or more low-energy conformers for each monomeric unit are selected from the energy ordered MMFF conformer list that are extended (this is necessary for polymerization to occur).

h) The can lining is treated as a supercooled liquid and the solubility in mole faction of solute/solvent is computed for each of the ingredients from the list in step e) in each of the can lining formulations using the standard solubility procedure in COSMOtherm with T=25°C, the solute treated as solid or liquid depending on its state in pure form at this temperature and the algorithm set to iterative;

i) The calculations in step h) are repeated at T=40°C;

j) The calculations in step h) are repeated at T=50°C;

k) The mole fractions at the 3 temperatures are screened for mousse formulation ingredients which are:

1. Only abundant in the can lining compositions which fail;

2. Show appreciable solubility at one or more of the 3 temperatures studied.

1) The ingredients that pass the screen in step k) are likely candidates for contributing to can corrosion during storage for these specific can liner formulations. An improved packaged mousse product can be made - such product can have improved shelf stability.

**Example 4:** Predicting mixed micelle size and shape to correlate viscosity A system is examined which consists of the three surfactants alkyl ethoxy sulphate (AES), alkyl poly-ethoxy alcohol (NI), and alkyl tri-methyl ammonium chloride (CxTMAC). Additionally, sodium chloride (NaCl) may be present. Viscosity is known for several systems with varying concentrations of NaCl, total surfactant concentration, and individual surfactant concentration. To predict viscosity, first the shape and size of the surfactant aggregates, or micelles are predicted using the molecular thermodynamic theory of surfactant self-assembly (as described in Langmuir 1991, 7, 2934-2969). For a given system, the individual concentration of each surfactant is known along with salt (NaCl) concentration. Surfactant structure parameters including chain length, head group size, and head group charge are used to predict the shape of the micelles for a given system and the number of surfactant molecules comprising the micelles. Micelle size and shape are predicted for several systems and compared with these systems' viscosities. A correlation is created, which can then be used to predict viscosities for new systems and thus result in improved products.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

All documents cited in the Detailed Description of the Invention are, in relevant part, incorporated herein by reference; the citation of any document is not to be construed as an admission that it is prior art with respect to the present invention. To the extent that any meaning or definition of a term in this document conflicts with any meaning or definition of the same term in a document incorporated by reference, the meaning or definition assigned to that term in this document shall govern.

While particular embodiments of the present invention have been illustrated and described, it would be obvious to those skilled in the art that various other changes and modifications can be made without departing from the spirit and scope of the invention. It is therefore intended to cover in the appended claims all such changes and modifications that are within the scope of this invention.

## Claims

1. A method of selecting and/or designing comprising

a.) calculating, for a system comprising at least one species, having at least one species characteristic, at least one thermodynamic property, physical property and/or molecular descriptor of said system using said at least one species characteristic; preferably said thermodynamic and/or physical property is selected from the group consisting of:

(i) system, phase, species and/or subspecies free energy;

(ii) system, phase, species and/or subspecies enthalpy;

(iii) species and/or subspecies chemical potential;

(iv) species and/or subspecies activity coefficient;

(v) the equilibrium concentration of a species;

(vi) pure species density;

(vii) pure species viscosity;

(viii) pure species vapor pressure;

(ix) species pKa;

(x) reaction free energy barrier;

(xi) spatial distribution of a species in an inhomogeneous phase;

(xii) species enthalpy of adsorption;

(xiii) species free energy of adsorption;

(xiv) polymer swelling degree; and/or

(xv) absorption, distribution, metabolism, excretion prediction; and said molecular descriptor is selected from the group consisting of;

(i) system, phase, and/or species sigma-moments;

(ii) system, phase, species and/or subspecies sigma profiles;

(iii) species and/or subspecies cavity volume;

(iv) species and/or subspecies cavity surface area;

(v) moments of cavity charge-density;

(vi) sigma-profile similarity index;

(vii) screening charge distribution based charged partial surface area descriptors; and/or

(viii) ligand scoring/fit functions; more preferably preferably said at least one species characteristic used to calculate said at least one thermodynamic property, physical property and/or molecular descriptor of said system comprises at least one species molecular state of said species; preferably said species molecular state comprises a total charge, an electronic state, and a molecular structure wherein said molecular structure comprises one or more elements independently selected from the group consisting of: number of atoms, atom types, and/or isotopes; mole fractions of atoms, atom types, and/or isotopes; molecular connectivity; and one or more sets of 3D atomic coordinates;

b.) optionally, using the thermodynamic property, physical property and/or molecular descriptor calculated in a.) to:

(i) calculate at least one additional thermodynamic property, physical property and/or molecular descriptor of said system; and/or

(ii) refine the thermodynamic property, physical property and/or molecular descriptor calculated in a.); and

c.) optionally repeating a.) through b.) one or more times, preferably steps a.) through c.) are repeated at least once; and

d.) using the thermodynamic property, physical property and/or molecular descriptor obtained from any of a.) through c.) to design and/or select a component for use in consumer product; design a consumer product; design and/or select a process for making said component and/or said consumer product, preferably d.) comprises comparing said thermodynamic property, physical property and/or molecular descriptor obtained from any of a.) through c.) with one or more additional thermodynamic properties, physical properties and/or molecular descriptors calculated in accordance with any of the calculation steps a.) through c.).

2. A method of selecting a design for and/or designing a consumer product or component thereof, or selecting a process and/or designing a process for making a consumer product or component thereof, said method comprising

a.) calculating, for a system comprising at least one species, having at least one species characteristic, at least one thermodynamic property, physical property and/or molecular descriptor of said system using said at least one species characteristic; preferably said thermodynamic and/or physical property is selected from the group consisting of:

(i) system, phase, species and/or subspecies free energy;

(ii) system, phase, species and/or subspecies enthalpy;

(iii) species and/or subspecies chemical potential;

(iv) species and/or subspecies activity coefficient;

(v) the equilibrium concentration of a species;
(vi) pure species density;
(vii) pure species viscosity;
(viii) pure species vapor pressure;
(ix) species pKa;
(x) reaction free energy barrier;
(xi) spatial distribution of a species in an inhomogeneous phase;
(xii) species enthalpy of adsorption;
(xiii) species free energy of adsorption;
(xiv) polymer swelling degree; and/or
(xv) absorption, distribution, metabolism, excretion prediction; and said molecular descriptor is selected from the group consisting of;
(i) system, phase, and/or species sigma-moments;
(ii) system, phase, species and/or subspecies sigma profiles;
(iii) species and/or subspecies cavity volume;
(iv) species and/or subspecies cavity surface area;
(v) moments of cavity charge-density;
(vi) sigma-profile similarity index;
(vii) screening charge distribution based charged partial surface area descriptors; and/or
(ix) ligand scoring/fit functions;

preferably said at least one species characteristic used to calculate said at least one thermodynamic property, physical property and/or molecular descriptor of said system comprises at least one species molecular state of said species; preferably said species molecular state comprises a total charge, an electronic state, and a molecular structure wherein said molecular structure comprises one or more elements independently selected from the group consisting of: number of atoms, atom types, and/or isotopes; mole fractions of atoms, atom types, and/or isotopes; molecular connectivity; and one or more sets of 3D atomic coordinates;
b.) optionally, using the thermodynamic property, physical property and/or molecular descriptor calculated in a.) to:

(iii) calculate at least one additional thermodynamic property, physical property and/or molecular descriptor of said system; and/or
(iv) refine the thermodynamic property, physical property and/or molecular descriptor calculated in a.); and

c.) optionally repeating a.) through b.) one or more times, preferably steps a.) through c.) are repeated at least once; and
d.) using the thermodynamic property, physical property and/or molecular descriptor obtained from any of a.) through c.) to design and/or select a component for use in consumer product; design a consumer product; design and/or select a process for making said component and/or said consumer product, preferably d.) comprises comparing said thermodynamic property, physical property and/or molecular descriptor obtained from any of a.) through c.) with one or more additional thermodynamic properties, physical properties and/or molecular descriptors calculated in accordance with any of the calculation steps a.) through c.).

3. The method of claim 1 or claim 2 comprising the additional step of making the consumer product or component thereof.

4. The method of claim 1 or claim 2 for designing and/or selecting a design for a consumer product or component thereof.

5. The method of claim 1 or claim 2 for designing and/or selecting a process for making a consumer product or component thereof.

6. The method of any preceding claim, comprising calculating using at least two system parameters of a system comprising at least one species and at least one phase, wherein at least one of said parameters is a species characteristic, at least one thermodynamic property, physical property and/or molecular descriptor of said system, preferably said calculation of said at least one thermodynamic property, physical property and/or molecular descriptor of said system is based on from 1 to 1000, from 1 to 500, or even from 2 to 150 system parameters, preferably the calculation of said at least one thermodynamic property, physical property and/or molecular descriptor of said system employs one or more system parameters selected from the group consisting of:

a.) a species molecular state;

b.) phase composition;

c.) system temperature;

d.) system pressure;

e.) system and/or phase external magnetic field;

f.) system and/or phase external electric field;

g.) system external gravitational field;

h.) phase dielectric constant;

i.) atomic/molecular cavity size;

j.) phase interface topology; and/or

k.) a species thermodynamic property; more preferably the calculation of said at least one thermodynamic property, physical property and/or molecular descriptor of said system employs one or more system parameters selected from the group consisting of:

a.) a species molecular state;

b.) phase composition;

c.) system temperature;

d.) phase dielectric constant;

e.) atomic/molecular cavity size; and/or

f.) a species thermodynamic property preferably said phase composition comprises one or more of the following elements: species mole fraction, species spatial probability, phase pH, phase ionic strength and said species thermodynamic property is selected from the group consisting of:

a.) free energy of phase change for a pure species;

b.) enthalpy of phase change for a pure species;

c.) entropy of phase change for a pure species;

d.) energy of phase change for a pure species;

e.) a pure species phase transition temperature;

f.) energy of pure species in vacuum;

g.) entropy of pure species in vacuum;

h.) a pure species vapor pressure;

i.) a pure phase constant pressure heat capacity; and

j.) a pure phase constant volume heat capacity. preferably the calculated at least one:

a.) thermodynamic and/or physical property is selected from the group consisting of:

    (i) system, phase, species and/or subspecies free energy;

    (ii) system, phase, species and/or subspecies enthalpy;

    (iii) species and/or subspecies chemical potential;

    (iv) species and/or subspecies activity coefficient;

    (v) the equilibrium concentration of a species;

    (vi) pure species density;

    (vii) pure species viscosity;

    (viii) pure species vapor pressure;

    (ix) species pKa;

    (x) reaction free energy barrier;

    (xi) spatial distribution of a species in an inhomogeneous phase;

    (xii) species enthalpy of adsorption;

    (xiii) species free energy of adsorption;

    (xiv) polymer swelling degree; and/or

    (xv) absorption, distribution, metabolism, excretion prediction;

b.) molecular descriptor is selected from the group consisting of;

    (i) system, phase, and/or species sigma-moments;

    (ii) system, phase, species and/or subspecies sigma profiles;

    (iii) species and/or subspecies cavity volume;

    (iv) species and/or subspecies cavity surface area;

    (v) moments of cavity charge-density;

    (vi) sigma-profile similarity index;

    (vii) screening charge distribution based charged partial surface area descriptors; and/or

(viii) ligand scoring/fit functions more preferably the calculated at least one:

a.) thermodynamic and/or physical property is selected from the group consisting of:

> (i) system, phase, species and/or subspecies free energy;
> (ii) system, phase, species and/or subspecies enthalpy;
> (iii) species and/or subspecies chemical potential;
> (iv) species and/or subspecies activity coefficient;
> (v) the equilibrium concentration of a species;
> (vi) pure species vapor pressure;
> (vii) species pKa;
> (viii) reaction free energy barrier;
> (ix) spatial distribution of a species in an inhomogeneous phase;
> (x) species enthalpy of adsorption;
> (xi) species free energy of adsorption; and/or
> (xii) absorption, distribution, metabolism, excretion prediction;

b.) molecular descriptor is selected from the group consisting of;

> (i) system, phase, and/or species sigma-moments;
> (ii) system, phase, species and/or subspecies sigma profiles;
> (iii) species and/or subspecies cavity volume;
> (iv) species and/or subspecies cavity surface area;
> (v) sigma-profile similarity index; and/or
> (vi) screening charge distribution based charged partial surface area descriptors most preferably the calculated at least one:

a.) thermodynamic and/or physical property is selected from the group consisting of:

> (i) system, phase, species and/or subspecies free energy;
> (ii) system, phase, species and/or subspecies enthalpy;
> (iii) species and/or subspecies chemical potential;
> (iv) the equilibrium concentration of a species;
> (v) pure species vapor pressure;
> (vi) species pKa;
> (vii) reaction free energy barrier; and/or
> (viii) spatial distribution of a species in an inhomogeneous phase;

b.) molecular descriptor is selected from the group consisting of;

> (i) system, phase, and/or species sigma-moments;
> (ii) system, phase, species and/or subspecies sigma profiles;
> (iii) species and/or subspecies cavity volume;
> (iv) species and/or subspecies cavity surface area; and/or
> (v) sigma-profile similarity index.

7. The method of any preceding claim comprising using the thermodynamic property, physical property and/or molecular descriptor calculated in a.) to calculate at least one thermodynamic property, physical property and/or molecular descriptor of said system, or to refine the thermodynamic property, physical property and/or molecular descriptor calculated in a.), preferably a.) through b.) is repeated one or more times.

8. The method of any of claims 1 to 6 comprising using the thermodynamic property, physical property and/or molecular descriptor calculated in a.) to:

> (i) calculate at least one additional thermodynamic property, physical property and/or molecular descriptor of said system; and/or
> (ii) refine the thermodynamic property, physical property and/or molecular descriptor calculated in a.).

9. The method of any of claims 1 to 6 wherein Step a.) comprises determining numerical parameters for at least one of said species, preferably said numerical parameters are selected from the group comprising screening charge density at dielectric boundary, histogram of surface area with respect to screening charge density, molecular surface, molecular volume, collision diameter, interaction energy, and/or optimized 3D structure; preferably such determination comprises obtaining existing numerical parameters and/or calculating said numerical parameters and, if said determination is not sufficient to produce said thermodynamic property, physical property and/or molecular descriptor;

a.) repeating such determination using an input comprising the results of said determination; and/or
b.) calculating, in accordance with a molecular thermodynamic theory, preferably said molecular thermodynamic theory is selected from the group consisting of COSMO-RS theory, SAFT theory, quasi-chemical theory, lattice fluid theory, mean-field theory, and combinations thereof; using an input comprising said numerical parameters, an output; and if said determination and/or calculation is not sufficient to produce said thermodynamic property, physical property and/or molecular descriptor;
c.) repeating one or more times, a.) and/or b.) using, said numerical parameters and/or said output.

10. A method according to any preceding claim in which maximum ingredient solubility in liquid laundry detergent is predicted and based on said prediction(s) desired ingredient(s) for incorporating into a liquid laundry detergent are selected, said ingredient preferably being a brightener, preferably comprising the additional step of making a liquid laundry detergent comprising the selected ingredient(s).

11. A method according to any preceding claim in which dissociation constants are predicted, said predictions are then used to generate representations of molecular structure, based on said representations, desired molecules for use in beauty care products are selected, said dissociation constants preferably being acid dissociation constants, said method preferably comprising the additional step of making a beauty care product comprising said selected molecules.

12. A method according to any preceding claim comprising predicting mixed micelle shape and size for a plurality of surfactant systems, correlating the mixed micelle shape and size with viscosity, predicting viscosity for new systems, selecting a new system with desired viscosity based on said predictions, said method preferably comprising the additional step of making a new detergent composition or surfactant system or component therefore.

13. A method according to any preceding claim in which one or more steps is carried out by a computer.

14. A component for use in consumer product, a consumer product, or process for making said component and/or said consumer product designed or selected in accordance with the method of any preceding claim.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5576282 A
- US 6306812 B1
- US 6326348 B1
- US 4430243 A
- US 5597936 A
- US 5595967 A
- WO 0032601 A
- US 6225464 B

- US 5879584 A
- US 5691297 A
- US 5574005 A
- US 5569645 A
- US 5565422 A
- US 5516448 A
- US 5489392 A
- US 5486303 A

**Non-patent literature cited in the description**

- **A. KLAMT ; F. ECKERT ; M. DIEDENHOFEN ; M. BECK.** First Principles Calculations of Aqueous pKa Values for Organic and Inorganic Acids Using COSMO-RS Reveal an Inconsistency in the Slope of the pKa Scale. *J. Phys. Chem. A,* 2003, vol. 107, 9380

- *Langmuir,* 1991, vol. 7, 2934-2969